# EUROPÄISCHE PATENTANMELDUNG

(11) **EP 2 881 134 A1**
(43) Veröffentlichungstag der Anmeldung: **10.06.2015**
(21) Anmeldenummer: 14195652.4
(22) Anmeldetag: 01.12.2014
(51) Int. Cl.: A61M 25/00

(54) **Katheterschlauchsystem sowie Verfahren zur Herstellung eines Katheterschlauchsystems**

(30) Priorität: 06.12.2013 DE 102013225154
(71) Anmelder: RAUMEDIC AG, 95213 Münchberg (DE)
(72) Erfinder: Gläsel, Björn, 95158 Kirchenlamitz (DE)
(74) Vertreter: Rau, Schneck & Hübner Patentanwälte Rechtsanwälte PartGmbB

(57) **Zusammenfassung**

Ein Katheterschlauchsystem hat einen ersten Schlauch, insbesondere einen Mehrlumenschlauch (1), und mindestens einen zweiten Schlauch, z. B. mindestens zwei Einlumenschläuche (2, 3). Die Innenlumen der beiden Schläuche stehen miteinander über eine ausgehärtete Verbindungsmasse in gegenüber einer Umgebung abgedichteter Fluidverbindung. Es resultiert ein Katheterschlauchsystem, bei dem die Schläuche sicher und dicht miteinander verbunden sind.

## Beschreibung

Die Erfindung betrifft ein Katheterschlauchsystem nach den Ansprüchen 1 und 2. Ferner betrifft die Erfindung ein Verfahren zur Herstellung eines derartigen Katheterschlauchsystems.

Bei der Herstellung von Kathetern besteht oftmals die Notwendigkeit, Katheterschlauchsysteme zu fertigen, bei denen Schläuche, insbesondere Mikroschläuche, verschiedener Durchmesser miteinander verbunden werden müssen bzw. bei denen Mehrlumenschläuche, insbesondere Mikro-Mehrlumenschläuche, mit zugehörigen Zuleitungsschläuchen verbunden werden müssen.

Es ist eine Aufgabe der vorliegenden Erfindung, derartige Katheterschlauchsysteme sowie ein Herstellungsverfahren hierfür anzugeben.

Diese Aufgabe ist erfindungsgemäß gelöst durch ein Katheterschlauchsystem mit den im Anspruch 1 angegebenen Merkmalen sowie durch ein Katheterschlauchsystem mit den im Anspruch 2 angegebenen Merkmalen. In Bezug auf das Herstellungsverfahren ist die Aufgabe erfindungsgemäß gelöst durch ein Verfahren nach Anspruch 8.

Es wurde erkannt, dass sich eine fluiddichte Verbindung zwischen den Lumen der Schläuche unterschiedlichen Innenlumen-Durchmessers oder zwischen dem Mehrlumenschlauch und den mehreren Einlumenschläuchen über eine aushärtbare Verbindungsmasse herstellen lässt. Hierüber lässt sich eine Fluidverbindung auch zwischen Mikroschläuchen herstellen, also zwischen Schläuchen mit einem Innenlumen mit einem Durchmesser von weniger als 0,5 mm, beispielsweise von 250 µm, von 150 µm, von 100 µm oder noch kleineren Innenlumen-Durchmessern. Die aushärtbare Verbindungsmasse stellt dabei die Dichtheit der Fluidverbindung sicher. Diese Herstellung der Fluidverbindung lässt sich einerseits zur Überbrückung von unterschiedlichen Innendurchmessern zu verbindender Schläuche und andererseits zur Verbindung eines Mehrlumenschlauchs mit einer entsprechenden Anzahl von Zuleitungsschläuchen vorteilhaft einsetzen.

Eine Verbindung dieser Vorteile ergibt sich bei einer Ausführung nach Anspruch 3.

Bei einer Ausführung nach Anspruch 4 wird beim gebrauchsfertigen Katheterschlauchsystem auf einen zusätzlichen Fluidverbindungskörper beim fertiggestellten Katheterschlauchsystem verzichtet. Dies ermöglicht Fluidverbindungen mit optimiertem Durchfluss.

Varianten für die Verbindungsmasse nach Anspruch 5 haben sich als besonders geeignet herausgestellt. Je nach den Anforderungen, die an eine Viskosität, an einen Aushärtezeitraum, an eine Aushärtetemperatur sowie an eine Materialverträglichkeit gestellt werden, kommt eine dieser Verbindungsmassen-Varianten oder eine Mischung hiervon zum Einsatz.

Ein Verbindungsschlauch nach Anspruch 6 muss nach einem Aushärten der Verbindungsmasse nicht aus dem Schlauchsystem gezogen werden, was die Herstellung vereinfacht.

Ein Verbindungsschlauch nach Anspruch 7 kann an die jeweiligen Innendurchmesser der zugehörigen Schlauchlumen angepasst sein. Der Verbindungsschlauch kann einen längs seiner Erstreckung variierenden Innendurchmesser aufweisen.

Die Vorteile eines Herstellungsverfahrens nach Anspruch 8 entsprechen denen, die vorstehend im Zusammenhang mit den erfindungsgemäßen Katheterschlauchsystemen bereits erläutert wurden.

Bei dem nach Anspruch 9 zu ziehenden Verbindungskörper handelt es sich seinerseits um einen Verbindungsschlauch. Dies erleichtert den Ziehvorgang, da ein zu ziehender Schlauch hinsichtlich seines Außendurchmessers nachgeben kann. Je nach den Anforderungen, die an das gebrauchsfertige Katheterschlauchsystem gestellt werden, kann entschieden werden, ob der Verbindungskörper gezogen wird oder nicht.

Ausführungsbeispiele der Erfindung werden nachfolgend anhand der Zeichnung näher erläutert. In dieser zeigen:
- Fig. 1: perspektivisch positioniert in einem Einlegekörper einen Zweilumenschlauch und zwei zugeordnete Einlumenschläuche, verbunden über einen Verbindungskörper, der in die Endabschnitte der jeweiligen, zugeordneten Schlauchlumen eindringt, vor einem Umgießen des Verbindungskörpers mit einer aushärtbaren Verbindungsmasse;
- Fig. 2: ausschnittsweise in einem axialen Längsschnitt eine weitere Ausführung einer vorpositionierten Anordnung eines Zweilumenschlauchs und zweier zugeordneter Einlumenschläuche mit eingeführten Verbindungskörpern vor dem Umgießen;
- Fig. 3: in einer zu Fig. 2 ähnlichen Darstellung eine weitere Ausführung der vorpositionierten Anordnung des Zweilumenschlauchs und zweier Einlumenschläuche mit jeweils als Verbindungsschlauch ausgeführtem Verbindungskörper;
- Fig. 4: in einer zu Fig. 2 ähnlichen Darstellung vorpositioniert einen Einlumenschlauch und einen zugeordneten weiteren Einlumenschlauch mit anderem Innendurchmesser mit zusätzlichem Verbindungskörper, der in die einander zugeordneten Endabschnitte der beiden Schläuche eindringt, wiederum vor dem Umgießen des Verbindungskörpers mit einer aushärtbaren Verbindungsmasse; und
- Fig. 5: in einer zu Fig. 4 ähnlichen Darstellung die beiden vorpositionierten Einlumenschläuche mit einem als Verbindungsschlauch ausgeführten Verbindungskörper.

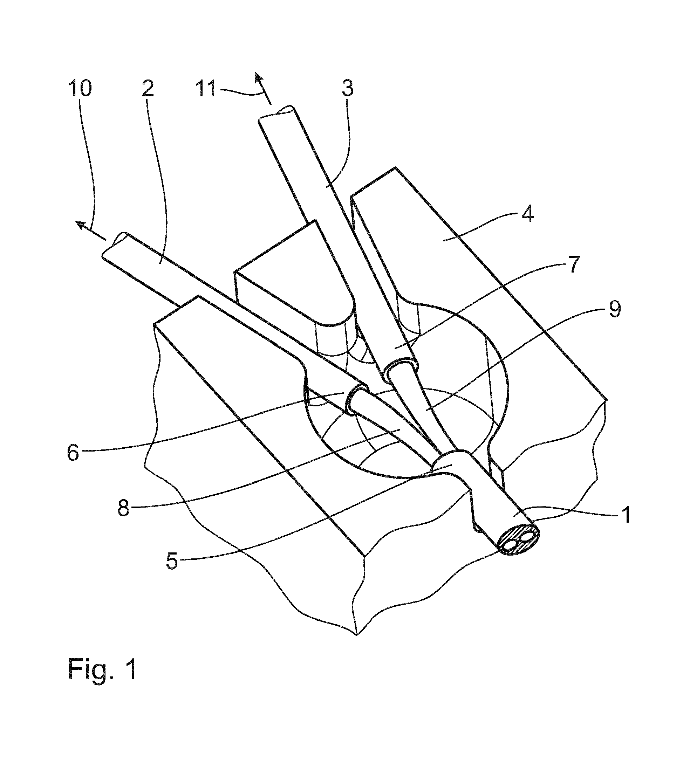

Fig. 1 zeigt eine Anordnung bei einem Fertigungsschritt eines Herstellungsverfahrens eines Katheterschlauchsystems. Zum fertigen Katheterschlauchsystem gehören ein Mehrlumenschlauch 1 in Form eines Zweilumenschlauchs, der einen ersten Schlauch darstellt, und zwei Einlumenschläuche 2, 3 jeweils als zweite Schläuche. Jeder der beiden Einlumenschläuche 2, 3 steht bei gefertigtem Katheterschlauchsystem in Fluidverbindung mit einem Lumen des Mehrlumenschlauches 1.

Im in Fig. 1 dargestellten Fertigungsschritt sind der Mehrlumenschlauch 1 und die beiden Einlumenschläuche 2, 3 in einem Einlegekörper 4, der gleichzeitig Bestandteil einer Gussform darstellt, so positioniert, dass ein Endabschnitt 5 des Mehrlumenschlauches 1 benachbart zu den korrespondierenden Endabschnitten 6, 7 der Einlumenschläuche 2, 3 angeordnet ist. Beim Einlegekörper 4 handelt es sich um eine Halbschale. Der Einlegekörper 4 kann ein Spritzguss-Bauteil darstellen.

Beim in der Fig. 1 dargestellten Fertigungsschritt sind zwei Verbindungskörper 8, 9 in die vorpositionierten Schläuche 1, 2 sowie 1, 3 eingeführt. Der Verbindungskörper 8 ist so in die vorpositionierten Schläuche 1, 2 eingeführt, dass der Verbindungskörper 8 einerseits in den Endabschnitt 5 des einen Lumens des Mehrlumenschlauches 1 und andererseits in den korrespondierenden Endabschnitt 6 des einen Einlumenschlauchs 2 eindringt. Der zweite Verbindungskörper 9 ist so vorpositioniert, dass dieser einerseits in den Endabschnitt 5 des anderen Lumens des Mehrlumenschlauchs 1 und andererseits in den korrespondierenden Endabschnitt 7 des anderen Einlumenschlauchs 3 eindringt.

Nach der Positionierung der Schläuche 1 bis 3 sowie der Verbindungskörper 8, 9 im Einlegekörper 4 gemäß Fig. 1 erfolgt ein Umgießen der Verbindungskörper 8, 9 im Bereich der gegenüberliegenden Endabschnitte 5, 6 und 7 mit einer aushärtbaren Verbindungsmasse. Bei der aushärtbaren Verbindungsmasse kann es sich um einen UV-Kleber, um ein Cyanacrylat, um einen Silikonklebstoff, um ein Epoxidharz, um einen Kraftkleber oder um ein Gemisch aus einem Klebstoff und einem Lösemittel handeln. Auch Verbindungsmassen, die aus mehreren dieser Bestandteile aufgebaut sind, sind möglich.

Die Verbindungsmasse wird anschließend ausgehärtet.

Zwei Varianten der Verbindungskörper 8, 9 sind möglich. Bei den Verbindungskörpern 8, 9 kann es sich um Mandrins handeln, die nach dem Aushärten der Verbindungsmasse aus dem Katheterschlauchsystem gezogen werden. In diesem Fall weisen die Verbindungskörper 8, 9 beispielsweise aus Draht gefertigte Endabschnitte auf, über die die Verbindungskörper 8, 9 in Richtung der in der Fig. 1 dargestellten Pfeile 10, 11 aus den Einlumenschläuchen 2, 3 gezogen werden können. Nach dem Ziehen der Mandrin-Verbindungskörper 8, 9 ist das Katheterschlauchsystem fertiggestellt, wobei die Innenlumen der Schläuche 1, 2 einerseits und der Schläuche 1, 3 andererseits miteinander über die ausgehärtete Verbindungsmasse in gegenüber einer Umgebung abgedichteter Fluidverbindung stehen. Der Einlumenschlauch 2 ist dabei mit einem der beiden Lumen des Mehrlumenschlauchs 1 und der andere Einlumenschlauch 3 ist mit dem anderen Lumen des Mehrlumenschlauchs 1 fluidverbunden, so dass die beiden Fluidverbindungen gegeneinander abgedichtet sind.

Bei der Ausführung der Verbindungskörper 8, 9 als Mandrins können diese als Vollkörper oder als Schlauchkörper ausgeführt sein.

Bei einer Variante des Herstellungsverfahrens sind die Verbindungskörper 8, 9 als Verbindungsschläuche ausgeführt. Nach dem Aushärten der Verbindungsmasse sind diese Verbindungsschläuche 8, 9 zwischen den Schläuchen 1, 2 einerseits und 1, 3 andererseits Bestandteil der jeweils abgedichteten Fluidverbindung.

Die Verbindungskörper 8, 9 haben längs ihrer Erstreckung einen variierenden Außendurchmesser, der an die Innendurchmesser der zugehörigen Schlauchlumen angepasst ist.

Die jeweiligen Innenlumen der Einlumenschläuche 2 und 3 sowie die Innenlumen des Mehrlumenschlauchs 1 haben einen Innendurchmesser im Bereich zwischen 0,1 mm und 5,0 mm. Diese Innendurchmesser können je nach den an das Katheterschlauchsystem gestellten Anforderungen unterschiedlich sein. So können sich die Innenlumen der beiden Einlumenschläuche 2, 3 voneinander und vom Innenlumen des Mehrlumenschlauchs 1 unterscheiden. Alternativ können auch mindestens zwei der Innenlumen der Schläuche 1 bis 3 den gleichen Innendurchmesser haben.

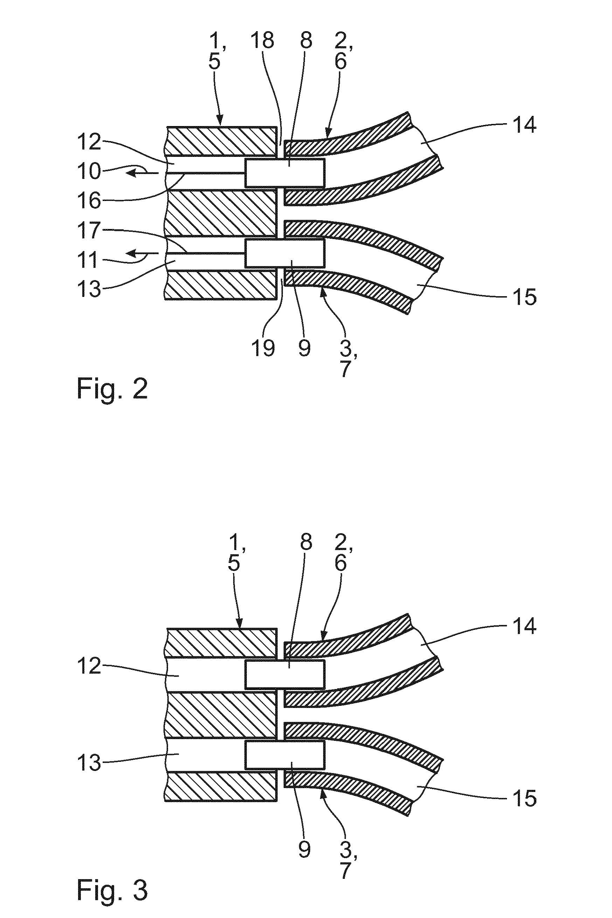

Anhand der Fig. 2 wird nachfolgend eine weitere Ausführung eines Katheterschlauchsystems beschrieben. Komponenten, die denjenigen entsprechen, die vorstehend unter Bezugnahme auf die Ausführung nach Fig. 1 bereits erläutert wurden, tragen die gleichen Bezugsziffern und werden nicht nochmals im Einzelnen diskutiert.

Bei der Ausführung nach Fig. 2 haben Innenlumen 12, 13 des Mehrlumenschlauches 1 den gleichen Innendurchmesser wie die Innenlumen 14, 15 der beiden Einlumenschläuche 2, 3. Die Verbindungskörper 8, 9 sind als Mandrins ausgeführt, die über Ziehdrähte 16, 17 gezogen werden können (vgl. Pfeile 10, 11). Beim Umgießen der Verbindungskörper 8, 9 wird jeweils ein Zwischenraum 18, 19 zwischen den Endabschnitten 5, 6 einerseits und den Endabschnitten 5, 7 andererseits der Lumen 12, 14 bzw. der Lumen 13, 15 fluiddicht zur Umgebung hin mit der ausgehärteten Verbindungsmasse verschlossen. Die Verbindungsmasse dringt beim Umgießen etwas in Zwischenräume zwischen den Verbindungskörpern 8, 9 einerseits und den Schlauch-Endabschnitten 5, 6 bzw. 5, 7 andererseits ein. Dieses Eindringen erfolgt über einen Weg, der kürzer ist als die Erstreckung der Verbindungskörper 8, 9 in die jeweiligen Endabschnitte 5, 6 bzw. 5, 7, so dass vermieden ist, dass die Verbindungsmasse die Lumen 12, 14 bzw. 13, 15 unerwünscht verschließt. Zudem gewährleistet dieses lediglich über einen kurzen Weg erfolgende Eindringen ein problemloses Ziehen der Verbindungskörper 8, 9 über die Ziehdrähte 16, 17. Um dieses einerseits geringe, andererseits sicher abdichtende Eindringen der Verbindungsmasse zu gewährleisten, werden einerseits die Weite der Eindring-Zwischenräume und andererseits die Viskosität der Verbindungsmasse sowie eine Eindringdauer vor dem Aushärten aufeinander abgestimmt.

Fig. 3 zeigt eine Variante der Verbindungskörper 8, 9 bei der Herstellung einer Variante eines Katheterschlauchsystems nach Fig. 2. Die Verbindungskörper 8, 9 sind bei der Ausführung nach Fig. 3 als Verbindungsschläuche ausgeführt, die nach dem Aushärten der Verbindungsmasse im Katheterschlauchsystem verbleiben, also Bestandteile der jeweils nach außen abgedichteten Fluidverbindung zwischen den Innenlumen 12, 14 bzw. zwischen den Innenlumen, 13, 15 sind. Die Verbindungsschläuche 8, 9 werden nach dem Aushärten der Verbindungsmasse also nicht gezogen.

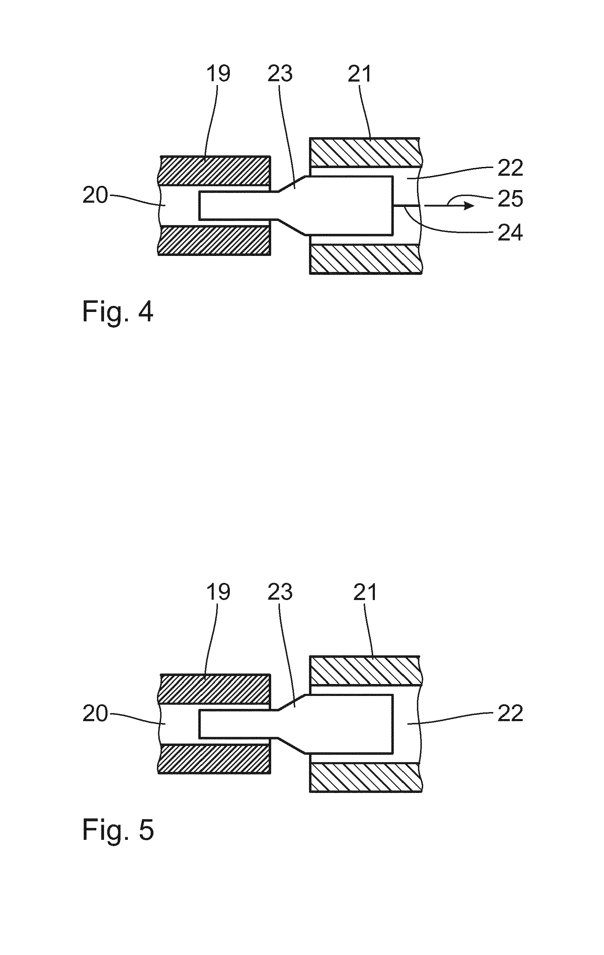

Anhand der Fig. 4 und 5 wird nachfolgend eine weitere Ausführung eines Katheterschlauchsystems beschrieben. Komponenten, die denjenigen entsprechen, die vorstehend unter Bezugnahme auf die Ausführungen nach den Fig. 1 bis 3 bereits erläutert wurden, tragen die gleichen Bezugsziffern und werden nicht nochmals im Einzelnen diskutiert.

Bei dem Katheterschlauchsystem nach Fig. 4 wird ein erster Schlauch 19 mit Innenlumen 20 mit einem zweiten Schlauch 21 mit Innenlumen 22 fluiddicht verbunden. Ein Durchmesser des Innenlumens 20 des ersten Schlauchs 19 ist kleiner als ein Durchmesser des Innenlumens 22 des zweiten Schlauchs 21. Bei der Herstellung des Kathetersystems werden Endabschnitte der beiden Schläuche 19, 21 in einem nicht dargestellten Einlegekörper in einer Stellung vorpositioniert, die derjenigen nach Fig. 4 entspricht. Ein Verbindungskörper 23, dessen Funktion derjenigen der Verbindungskörper 8, 9 der Ausführungen nach den Fig. 1 bis 4 entspricht, wird in die vorpositionierten Schläuche 19, 21 eingeführt, so dass der Verbindungskörper 23 in einander zugewandte Endabschnitte der beiden Schläuche 19, 21 endseitig eindringt. Der Verbindungskörper hat einen längs seiner Erstreckung variierenden Außendurchmesser, der jeweils an den Innendurchmesser der Lumen 20, 22 der beiden Schläuche 19, 21 angepasst ist. Im Bereich des Endabschnitts 19 hat der Verbindungskörper 23 also einen kleineren Außendurchmesser als im Bereich des Endabschnitts des zweiten Schlauchs 21.

In der Relativposition des Verbindungskörpers 23 zu den beiden Schläuchen 19, 21, die in Fig. 4 dargestellt ist, wird sodann der Verbindungskörper 23 mit der aushärtbaren Verbindungsmasse umgossen und die Verbindungsmasse wird ausgehärtet. Anschließend wird der Verbindungskörper, der als Mandrin ausgeführt ist, über einen Ziehdraht 24 in Richtung des Pfeils 25 gezogen, entsprechend dem, was vorstehend im Zusammenhang mit den Fig. 1 und 2 bereits ausgeführt wurde.

Beim Katheterschlauchsystem nach Fig. 5 ist der Verbindungskörper 23 als Verbindungsschlauch ausgeführt, der Bestandteil der abgedichteten Fluidverbindung zwischen den Lumen 20, 22 ist, die nach dem Aushärten der Verbindungsmasse entstanden ist. Der Verbindungsschlauch 23 nach Fig. 5 wird nach dem Umgießen und Aushärten der Verbindungsmasse also nicht gezogen, sondern verbleibt zwischen den einander zugewandten Endabschnitten der Schläuche 19 und 21.

Auch dann, wen die Verbindungskörper 8, 9 bzw. 23 nach dem Aushärten der Verbindungsmasse gezogen werden, können die Verbindungskörper 8, 9 bzw. 23 als Schläuche ausgeführt sein, da dies das Ziehen der Verbindungskörper 8, 9 bzw. 23 erleichtert.

## Patentansprüche

1. Katheterschlauchsystem
- mit einem ersten Schlauch (19),
- mit mindestens einem zweiten Schlauch (21) mit mindestens einem Innenlumen (22), dessen Durchmesser sich vom Durchmesser des mindestens einen Innenlumens (20) des ersten Schlauchs (19) unterscheidet,
- wobei die Innenlumen (20, 22) der beiden Schläuche miteinander über eine ausgehärtete Verbindungsmasse in gegenüber einer Umgebung abgedichteter Fluidverbindung stehen.

2. Katheterschlauchsystem
- mit einem Mehrlumenschlauch (1) als ersten Schlauch,
- mit mindestens zwei Einlumenschläuchen (2, 3) als zweiten Schläuchen,
- wobei jeder der Einlumenschläuche (2, 3) in Fluidverbindung mit einem Lumen (12, 13) des Mehrlumenschlauchs (1) steht,
- wobei die Fluidverbindungen zwischen den Lumen (12, 13) des Mehrlumenschlauchs (1) und den Einlumenschläuchen (2, 3) fluiddicht über eine ausgehärtete Verbindungsmasse jeweils gegeneinander abgedichtet sind.

3. Katheterschlauchsystem nach Anspruch 2, **dadurch gekennzeichnet, dass** sich der Durchmesser mindestens eines der Innenlumen der Einlumenschläuche (2, 3) sich vom Durchmesser des zugehörigen Innenlumens des Mehrlumenschlauchs (1) unterscheidet.

4. Katheterschlauchsystem nach einem der Ansprüche 1 bis 3, **dadurch gekennzeichnet, dass** die mindestens eine abgedichtete Fluidverbindung vollständig über eine ausgehärtete Verbindungsmasse gegeben ist.

5. Katheterschlauchsystem nach einem der Ansprüche 1 bis 4, **gekennzeichnet durch** eine ausgehärtete Verbindungsmasse aus mindestens einem der folgenden Materialien:
- UV-Kleber,
- Cyanacrylat,
- Silikonklebstoff,
- Epoxidharz,
- Kraftkleber,
- Gemisch aus Lösemittel und Klebstoff.

6. Katheterschlauchsystem nach einem der Ansprüche 1 bis 3 und 5, **gekennzeichnet durch** einen Verbindungsschlauch (8, 9; 23) zwischen den Schläuchen (1,2; 1,3; 19, 21), der Bestandteil der abgedichteten Fluidverbindung ist.

7. Katheterschlauchsystem nach Anspruch 6, **dadurch gekennzeichnet, dass** der Verbindungsschlauch (8, 9; 23) einen längs seiner Erstreckung variierenden Außendurchmesser aufweist.

8. Verfahren zur Herstellung eines Katheterschlauchsystems nach einem der Ansprüche 1 bis 7 mit folgenden Schritten:
- Positionieren des ersten Schlauchs (1; 19) und des mindestens einen zweiten Schlauchs (2, 3; 21), so dass ein Endabschnitt (5) des mindestens einen Lumens (12, 13; 20) des ersten Schlauchs (1; 19) benachbart zum korrespondierenden Endabschnitt (6, 7) des Lumens (14, 15; 22) des mindestens einen zweiten Schlauchs (2, 3; 21) angeordnet ist,
- Einführen mindestens eines als Verbindungsschlauch ausgeführten Verbindungskörpers (8, 9; 23) in die vorpositionierten Schläuche (1, 2, 3; 19, 21) derart, dass der Verbindungskörper (8, 9; 23) einerseits in den Endabschnitt (5) eines der Lumen (12, 13; 20) des ersten Schlauchs (1; 19) und andererseits in den korrespondierenden Endabschnitt (6, 7) des zweiten Schlauchs (2, 3; 21) eindringt,
- Umgießen des Verbindungskörpers (8, 9; 23) im Bereich der beiden gegenüberliegenden Endabschnitte (5, 6, 7) mit einer aushärtbaren Verbindungsmasse,
- Aushärten der Verbindungsmasse zur Erzeugung der ausgehärteten Verbindungsmasse.

9. Verfahren nach Anspruch 8, **dadurch gekennzeichnet, dass** der Verbindungskörper (8, 9; 23) nach dem Aushärten der Verbindungsmasse aus dem Katheterschlauchsystem gezogen wird.
